# EUROPEAN PATENT SPECIFICATION

(11) **EP 3 389 640 B1**
(45) Date of publication and mention of the grant of the patent: **18.03.2020**
(21) Application number: 16822935.9
(22) Date of filing: 16.12.2016
(51) Int. Cl.: A61K 9/20, A61K 9/28, A61K 31/506

(54) **PHARMACEUTICAL COMPOSITION COMPRISING ANHYDROUS DASATINIB**
PHARMAZEUTISCHE ZUSAMMENSETZUNG MIT WASSERFREIEM DASATINIB
COMPOSITION PHARMACEUTIQUE COMPRENANT DU DASATINIB ANHYDRE

(30) Priority: 16.12.2015 EP 15200445
(43) Date of publication of application: 24.10.2018
(73) Proprietor: Synthon B.V., 6545 CM Nijmegen (NL)
(72) Inventor: GARCIA JIMENEZ, Sonia, 08830 Sant Boi de Llobregat (ES); ALVAREZ FERNANDEZ, Lisardo, 08830 Sant Boi de Llobregat (ES); VELADA CALZADA, Jose, 6545 CM Nijmegen (NL)
(74) Representative: Overeem, Arjanne
(86) International application number: PCT/EP2016/081367
(87) International publication number: WO 2017/103057

(56) References cited:
- CN-A- 104 274 420
- CN-B- 102 836 159
- US-B2- 7 973 045

## Description

### BACKGROUND OF THE PRESENT INVENTION

Dasatinib, chemically *N*-(2-chloro-6-methylphenyl)-2-[[6-[4-(2-hydroxyethyl)-1-piperazinyl]-2-methyl-4-pyrimidinyl]amino]-5-thiazolecarboxamide of formula (I), 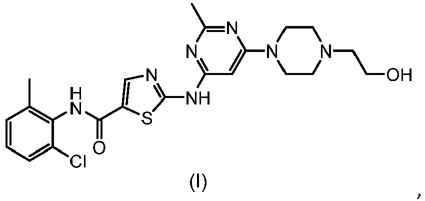 is a pharmaceutically active compound used for the treatment of adult patients with chronic myelogenous leukaemia (CML) after imatinib treatment, Philadelphia chromosome-positive acute lymphoblastic leukaemia (ALL) and newly diagnosed Philadelphia chromosome positive (Ph+) chronic myelogenous leukaemia (CML) in the chronic phase.

Dasatinib was discovered by Bristol-Myers Squibb and is disclosed in EP1169038. Dasatinib is the active ingredient in the medicinal product sold under the brand name Sprycel®.

Several crystalline forms of dasatinib are known and described in literature. WO2005077945 discloses a crystalline monohydrate of dasatinib and a butanol solvate of dasatinib. The marketed product Sprycel® contains the crystalline monohydrate of dasatinib. WO2005077945 further discloses a crystalline ethanol solvate and two anhydrous forms of dasatinib. Other forms of dasatinib are disclosed in WO2009053854, WO2010062715, WO2010067374, WO2011095059 and WO2012014149. Some of the described forms do contain unwanted solvents. Moreover, it was experienced in our laboratory that, especially under humid conditions, some of these forms are rather unstable.

Dasatinib monohydrate is a BCS class II compound, exhibiting low solubility and high permeability. Its low aqueous solubility affects the dissolution behavior of dasatinib monohydrate negatively.

Thus in view of the prior art cited above, there is still a need for a stable pharmaceutical compositions with adequate dissolution comprising dasatinib, which is suitable for production on commercial scale.

### BRIEF DESCRIPTION OF THE PRESENT INVENTION

The present invention provides a tablet composition comprising anhydrous dasatinib and one or more pharmaceutically acceptable excipients, wherein the X-ray powder diffraction pattern of anhydrous dasatinib comprises characteristic peaks at the following 2 theta (± 0.2) angles: 6.8°, 11.1°, 12.3°, 13.2°, 13.7°, 16.7°, 21.0°, 24.3° and 24.8°, measured using a Cu Kα radiation and wherein the tablet is film coated with a coating composition comprising triacetin as plasticizer.

It also provides a process for preparing the tablet composition comprising granulation.

Said pharmaceutical composition may be used as a medicament, particularly in the treatment of chronic myeloid leukaemia (CML) and acute lymphoblastic leukaemia (ALL).

### BRIEF DESCRIPTION OF THE DRAWINGS

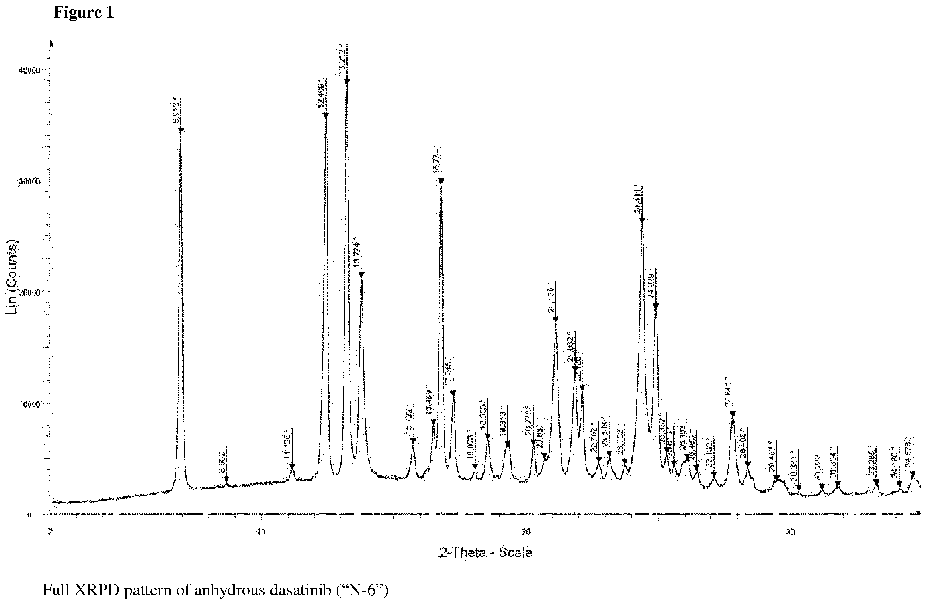

Figure 1 shows the full XRPD pattern of anhydrous dasatinib ("N-6"). For measurement conditions see the Examples section.

### DETAILED DESCRIPTION OF THE PRESENT INVENTION

Sprycel® contains the monohydrated form of dasatinib as disclosed in WO2005077945, the so-called form "H1-7". Dasatinib monohydrate is a very stable compound, but being a BCS class II compound, it exhibits low aqueous solubility which affects dissolution behavior.

Other forms of dasatinib have been disclosed in the prior art. Many of these forms are solvated forms of dasatinib and some of them do contain unwanted (e.g. toxic) solvents. Moreover, some of the forms as disclosed in the prior art are rather unstable.

WO2005077945 discloses, besides dasatinib monohydrate, crystalline butanol and ethanol solvates and two anhydrous forms of dasatinib. One of the anhydrous forms of dasatinib is the so-called "N-6" form. The X-ray powder diffraction pattern of this form comprises characteristic peaks at the following 2 theta (± 0.2) angles: 6.8°, 11.1°, 12.3°, 13.2°, 13.7°, 16.7°, 21.0°, 24.3° and 24.8°, measured using a Cu Kα radiation. The XRPD pattern of anhydrous dasatinib form "N-6" is shown in figure 1. This anhydrous form is less stable than dasatinib monohydrate, but exhibits a significantly higher aqueous solubility when compared to dasatinib monohydrate. The solubility of anhydrous form "N-6" at pH 1.2 is 34.51 mg/ml, while at the same pH value the solubility of dasatinib monohydrate "H1-7" is only 2.85 mg/ml. It would thus be advantageous to use this anhydrous form in a pharmaceutical composition.

EP1885339 discloses pharmaceutical compositions comprising dasatinib and a nonreactive coating having polyethylene glycol as plasticizer. Commonly used plasticizers in coating formulations can be categorized into three groups: (1) Polyols (glycerin, propylene glycol, polyethylene glycols); (2) Organic esters (phthalate esters, dibutyl sebacate, citrate esters, triacetin); (3) Oils/glycerides (castor oil, acetylated monoglycerides, fractionated coconut oil).

The examples disclosed in EP1885339 were carried out with the composition comprising monohydrate of dasatinib. During prosecution of EP 1885339, the patentee showed that tablets comprising dasatinib monohydrate coated with a film coating containing polyethylene glycol as plasticizer provide an improvement in chemical stability of dasatinib in comparison to other plasticizers. Experimental data was presented wherein tablets coated with two tablet film coating formulations were studied: one formulation containing polyethylene glycol as plasticizer, the other formulation having triacetin as plasticizer. After storage at elevated temperature or increased relative humidity, it was found that triacetin reacted with dasatinib and that the total degradation products of dasatinib in the tablet was found to be significantly lower using a film coat containing polyethylene glycol as plasticizer.

The tablet composition of the present invention comprises anhydrous dasatinib form "N-6". It was found that the tablet core comprising dasatinib "N-6" is prone to oxidation. After 6 months at 40°C/75% RH, the uncoated tablets contain a significant amount of the N-oxide (see table 4). This oxidation can be prevented by coating the tablets with a suitable coating formulation. Very surprisingly it was found that by using anhydrous dasatinib form "N-6", the chemical stability of tablets coated with a film coating formulation comprising triacetin as plasticizer is excellent and that oxidation of dasatinib "N-6" is suppressed successfully. Unlike dasatinib monohydrate, anhydrous dasatinib "N-6" does not react with the organic ester triacetin when used as plasticizer in the coating formulation.

The present invention provides a tablet composition comprising anhydrous dasatinib and one or more pharmaceutically acceptable excipients, wherein the X-ray powder diffraction pattern of anhydrous dasatinib comprises characteristic peaks at the following 2 theta (± 0.2) angles: 6.8°, 11.1°, 12.3°, 13.2°, 13.7°, 16.7°, 21.0°, 24.3° and 24.8°, measured using a Cu Kα radiation and wherein the tablet is film coated with a coating composition comprising triacetin as plasticizer.

In addition to triacetin, the coating composition of the present invention comprises a polymer and a pigment.

The polymer to be used in accordance with the present invention may be any polymer known to a person of ordinary skill in the art, but is preferably a cellulose polymer. The cellulose polymer used in the tablet coating composition may be selected from the group consisting of methylcellulose, hydroxypropylcellulose (HPC), hydroxypropylmethylcellulose (HPMC), hydroxyethylcellulose (HEC), hydroxyethylmethylcellulose (HEMC) or a combination thereof. HPMC is a particularly preferred cellulose polymer.

The pigment to be used in accordance with the present invention may be any pigment known to the person of ordinary skill in the art. Most preferred pigment is titanium dioxide.

Furthermore, the coating formulation of the present invention may contain a compound to improve polymer adhesion. A particularly preferred example of such a compound is lactose.

A typical example of a commercially available coating composition in accordance with the present invention is Opadry® II White.

The tablet compositions of the present invention display dissolution behavior typical for immediate-release formulations and mimic the dissolution profile of the Sprycel® tablets. The compositions of the present invention exhibit a dissolution rate of at least 85% in 20 minutes when tested in 500 ml aqueous hydrochloric acid (0.01 N) pH 2.0 or of at least 40% in 10 minutes and 60% in 30 minutes when tested in 900 ml acetate buffer with 0.4% Tween 80 pH 4.5 in a USP apparatus II at 25°C, 75 rpm.

The tablet compositions according to the present invention comprise, besides anhydrous dasatinib, one or more pharmaceutically acceptable excipients. The excipients to be used in accordance with the present invention are well-known and are those excipients which are conventionally used by the person skilled in the art. The pharmaceutically acceptable excipients are chosen from one or more binders, diluents, disintegrants, glidants or lubricants.

The diluent to be used in accordance with the present invention may be any diluent known to a person of ordinary skill in the art. Particularly, the diluent to be used in accordance with the present invention is an inorganic diluent, polysaccharide, mono- or disaccharide or sugar alcohol. Microcrystalline cellulose and lactose are particularly preferred diluents.

The binder to be used in accordance with the present invention may be any binder known to a person of ordinary skill in the art. Suitable binders are selected from the group consisting of hydroxypropyl methylcellulose, hydroxypropylcellulose, hydroxyethylcellulose, sodium alginate, xanthan gum and sodium carboxymethylcellulose. Hydroxypropylcellulose is a particularly preferred binder.

The disintegrant to be used in accordance with the present invention may be any disintegrant known to a person of ordinary skill in the art. Suitable disintegrants to be used in accordance with the present invention are selected from the group consisting of croscarmellose sodium, crospovidone or sodium starch glycolate. Croscarmellose sodium is a particularly preferred disintegrant.

The glidant to be used in accordance with the present invention may be any glidant known to a person of ordinary skill in the art. Colloidal silicon dioxide is a particularly preferred glidant.

The lubricant to be used in accordance with the present invention may be any lubricant known to a person of ordinary skill in the art. Magnesium stearate is a particularly preferred lubricant.

The present invention further provides a process to prepare a tablet composition comprising anhydrous dasatinib and one or more pharmaceutically acceptable excipients comprising granulation. The granulation processes applied are simple and cost effective and include a standard wet or dry granulation technique.

The wet granulation process is performed with a granulation solvent selected from the group consisting of water, acetone, ethanol, isopropanol or a mixture thereof. The most preferred solvent to be used in accordance with the present invention is water. Very surprisingly, dasatinib anhydrous does not convert into dasatinib monohydrate during the process of granulation in water.

The dry granulation process is conducted by either slugging or roller compaction.

Preferably, the tablet prepared by wet granulation comprises, besides dasatinib anhydrous, one or more pharmaceutically acceptable binders, diluents, disintegrants and lubricants. Most preferably, the tablet prepared by using a wet granulation process comprises dasatinib anhydrous, lactose, microcrystalline cellulose, hydroxypropylcellulose, croscarmellose sodium and magnesium stearate. The binder is added to the intragranular phase as solid material or as solution in e.g. water. In a preferred embodiment of the present invention, the binder is added to the intragranular phase as solid material, which is more efficient by saving the step of dissolving. The disintegrant can be added as intragranular or extragranular component or it can be divided over the intragranular and extragranular phase. The diluent can be added as intragranular component or it can be divided over the intragranular and extragranular phase. In a preferred embodiment, the diluent is added partially to the intragranular phase and partially to the extragranular phase. The lubricant is added as extragranular component.

Preferably, the tablet prepared by dry granulation comprises, besides dasatinib anhydrous, one or more pharmaceutically acceptable diluents, disintegrants, lubricants and glidants. Most preferably, the tablet prepared by using a dry granulation process comprises dasatinib anhydrous, lactose, microcrystalline cellulose, croscarmellose sodium, magnesium stearate and colloidal silicon dioxide. The grade of lactose used in the dry granulation process is preferably lactose anhydrous, since its brittle nature allows recompaction without loss of tabletabillity. The disintegrant can be added as intragranular component or it can be divided over the intragranular and extragranular phase. In a preferred embodiment, the disintegrant is added partially to the intragranular phase and partially to the extragranular phase. The diluent can be added as intragranular component or it can be divided over the intragranular and extragranular phase. In a preferred embodiment, the diluent is added partially to the intragranular phase and partially to the extragranular phase. The lubricant is added partially intragranularly and partially extragranularly as well, while the glidant is added to the intragranular phase.

The tablet composition in accordance with the present invention may be used as a medicament. The composition typically may be used in the treatment of chronic myeloid leukaemia (CML) and acute lymphoblastic leukaemia (ALL).

The following examples are intended to illustrate the scope of the present invention but not to limit it thereto.

### EXAMPLES

### Example 1: anhydrous dasatinib "N-6"

The full XRPD pattern of anhydrous dasatinib ("N-6") of Figure 1 was obtained using a Bruker-AXS D8 Vario diffractometer with θ/2θ geometry (reflection mode), equipped with a Vantec PSD detector and applying the following measurement conditions:
- Start angle (2θ): 2.0°;
- End angle (2θ): 35.0°;
- Scan step width: 0.02°;
- Scan step time: between 0.2-2.0 seconds;
- Radiation type: Cu;
- Radiation wavelengths: 1.5406Å (Kα1), primary monochromator used;
- Exit slit: 6.0 mm;
- Focus slit: 0.2 mm;
- Divergence slit: Variable (V20);
- Antiscatter slit: 11.8 mm;
- Receiving slit: 20.7 mm.

Table 1 gives an overview of the solubility of anhydrous dasatinib ("N-6") and dasatinib monohydrate ("H1-7") at different pH values.

**Table 1: solubility of anhydrous dasatinib ("N-6") and dasatinib monohydrate ("H1-7")**

| | **pH 1.2** | **pH 4.5** | **pH 6.8** |
|---|---|---|---|
| Anhydrous dasatinib ("N-6") | 34.51 mg/ml | 0.179 mg/ml | 0.0018 mg/ml |
| Dasatinib monohydrate ("H1-7") | 2.85 mg/ml | 0.134 mg/ml | 0.0006 mg/ml |

### Example 2: tablet composition A comprising anhydrous dasatinib, prepared by wet granulation process

The tablets comprising anhydrous dasatinib prepared by wet granulation have the composition A as given in table 2.

**Table 2: tablet composition A of tablets prepared by wet granulation process**

| **Component** | **Quantity (mg/tablet)** | **Weight%** |
|---|---|---|
| *Intragranular components* | | |
| Anhydrous dasatinib | 140.00 | 25.00 |
| Lactose monohydrate | 189.00 | 33.75 |
| Microcrystalline cellulose | 160.65 | 28.69 |
| Hydroxypropyl cellulose | 16.80 | 3.00 |
| Croscarmellose sodium | 11.20 | 2.00 |
| Purified water | | q.s. |

| *Extragranular components* | | |
|---|---|---|
| Microcrystalline cellulose | 28.35 | 5.06 |
| Croscarmellose sodium | 11.20 | 2.00 |
| Magnesium stearate | 2.80 | 0.50 |
| **Total core tablet weight** | **560.00** | **100.00** |

The intragranular components were blended with water in a high shear mixer. The resulting granulate was dried in a fluid bed dryer. The granulate was sieved through an appropriate mesh size sieve. Microcrystalline cellulose and croscarmellose sodium were sieved through a suitable mesh size sieve for deagglomeration. The excipients were mixed with the sieved granulate in a diffusion mixer. Magnesium stearate was sieved through an appropriate sieve to deagglomerate and mixed with the powder mix in a diffusion mixer. The homogeneous powder obtained, was compressed using a rotating tablet press using appropriate punches. Part of the tablets were coated with a water suspension of Opadry® II White 31K28459 coating until 3% weight gain (= 16.8 mg/140 mg tablet). The tablets were packed in suitable packaging material and were stored at 40°C/75% RH.

### Stability results:

**Table 3: Stability results at 40°C/75% RH for uncoated tablets (composition A) prepared by wet granulation stored in HDPE open bottles**

| **Test** | **t=0** | **t= 6 months 40°C/75%RH** |
|---|---|---|
| Assay (%) | 98.5 | 98.8 |
| Impurities (%) | | |
| Impurity A | N.D. | <0.05 |
| Impurity B | <0.05 | 0.13 |
| Impurity C | N.D. | 0.05 |
| Impurity D | N.D. | <0.05 |
| Single largest unspecified | N.D. | 0.15 |
| Total unspecified impurities/ degradants | N.D. | 0.15 |
| | **<0.05%** | **0.33** |
| **Total impurities/degradants** | | |

**Table 4: Chemical structures of different impurities**

| **Impurity** | **Chemical structure** |
|---|---|
| Impurity A | 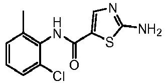 |
| Impurity B | 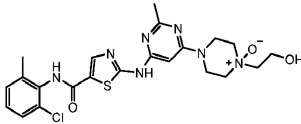 |
| Impurity C | 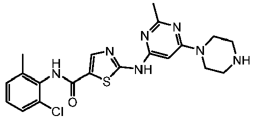 |
| Impurity D | 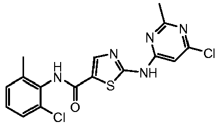 |

**Table 5: Stability results at 40°C/75% RH for coated tablets (composition A) prepared by wet granulation stored in different packaging materials**

| | | **t= 6 months 40°C/75% RH** | | |
|---|---|---|---|---|
| **Test** | **t= 0** | **HDPE open bottle** | **HDPE bottle/PP cap** | **Alu/Alu blister** |
| Assay (%) | 97.9 | 98.7 | 98.1 | 98.3 |
| Impurities (%) | | | | |
| Impurity A | N.D. | <0.05 | N.D. | N.D. |
| Impurity B | <0.05 | <0.05 | <0.05 | <0.05 |
| Impurity C | N.D. | <0.05 | <0.05 | <0.05 |
| Impurity D | N.D. | N.D. | N.D. | N.D. |
| Single largest unspecified | N.D. | 0.05 | <0.05 | <0.05 |
| Total unspecified impurities/ degradants | N.D. | 0.05 | <0.05 | <0.05 |
| **Total impurities/degradants** | **<0.05** | **0.05** | **<0.05** | **<0.05** |

XRPD analysis performed after storing the tablets in several types of packaging material (HDPE open bottle, HDPE bottle/PP cap, Alu/Alu blister) for 6 months at 40°C/75% RH showed only reflections in accordance with anhydrous dasatinib.

### Example 3: tablet composition B comprising anhydrous dasatinib, prepared by wet granulation process

The tablets comprising anhydrous dasatinib prepared by wet granulation have the composition B as given in table 6.

**Table 6: tablet composition B of tablets prepared by wet granulation process**

| **Component** | **Quantity (mg/tablet)** | **Weight%** |
|---|---|---|
| *Intragranular components* | | |
| Anhydrous dasatinib | 140.00 | 25.00 |
| Lactose monohydrate | 189.00 | 33.75 |
| Microcrystalline cellulose | 155.05 | 27.69 |
| Hydroxypropyl cellulose | 22.40 | 4.00 |
| Purified water | | q.s. |

| *Extragranular components* | | |
|---|---|---|
| Microcrystalline cellulose | 39.55 | 7.06 |
| Croscarmellose sodium | 11.20 | 2.00 |
| Magnesium stearate | 2.80 | 0.50 |
| **Total core tablet weight** | **560.00** | **100.00** |

Hydroxypropyl cellulose was dissolved in water and blended with the other intragranular components in a high shear mixer. The resulting granulate was dried in a fluid bed dryer. The granulate was sieved through an appropriate mesh size sieve. Microcrystalline cellulose and croscarmellose sodium were sieved through a suitable mesh size sieve for deagglomeration. The excipients were mixed with the sieved granulate in a tumbling mixer. Magnesium stearate was sieved through an appropriate sieve to deagglomerate and mixed with the powder mix in a tumbling mixer. The homogeneous powder obtained, was compressed using a rotating tablet press using appropriate punches. Part of the tablets were coated with a water suspension of Opadry® II White 31K28459 coating until 3% weight gain (= 16.8 mg/140 mg tablet). The tablets were packed in suitable packaging material and were stored at 40°C/75% RH.

**Table 7: Stability results at 40°C/75% RH for coated tablets (composition B) prepared by wet granulation stored in different packaging materials**

| | | **t= 1 month 40°C/75% RH** | |
|---|---|---|---|
| **Test** | **t= 0** | **HDPE bottle/PP cap** | **Alu/Alu blister** |
| Assay (%) | 102.3 | 99.9 | 101.4 |
| Impurities (%) | | | |
| Single largest unspecified impurities/ degradants | <0.1 | <0.1 | <0.1 |
| **Total impurities/degradants** | **<0.1** | **<0.1** | **<0.1** |

XRPD analysis performed after storing the tablets in several types of packaging material (HDPE open bottle, HDPE bottle/PP cap, Alu/Alu blister) for 1 month at 40°C/75% RH showed only reflections in accordance with anhydrous dasatinib.

### Example 4: composition C comprising anhydrous dasatinib, prepared by dry granulation process

The tablets comprising anhydrous dasatinib prepared by dry granulation have the composition C as given in table 8.

**Table 8: tablet composition C of tablets prepared by dry granulation process**

| **Component** | **Quantity (mg/tablet)** | **Weight%** |
|---|---|---|
| *Intragranular components* | | |
| Anhydrous dasatinib | 140.00 | 25.00 |
| Colloidal silicon dioxide | 2.80 | 0.50 |
| Lactose anhydrous | 194.60 | 34.75 |
| Microcrystalline cellulose | 165.41 | 29.54 |
| Croscarmellose sodium | 11.20 | 2.00 |
| Magnesium stearate | 2.80 | 0.5 |

| *Extragranular components* | | |
|---|---|---|
| Microcrystalline cellulose | 29.19 | 5.21 |
| Croscarmellose sodium | 11.20 | 2.00 |
| Magnesium stearate | 2.80 | 0.5 |
| **Total core tablet weight** | **560.00** | **100.00** |

Anhydrous dasatinib and colloidal silicon dioxide were sieved through an appropriate mesh size sieve and blended in a diffusion mixer to obtain a "pre-blend". Lactose anhydrous, microcrystalline cellulose and croscarmellose sodium were sieved through an appropriate mesh size sieve and mixed with the "pre-blend" in a diffusion mixer to obtain a "pre-lubricated blend". Magnesium stearate was sieved through an appropriate mesh size sieve and blended with the "pre-lubricated blend" in a diffusion mixer. The obtained lubricated blend was compressed into slugs and subsequently sieved using a conical sieving machine. The extragranular microcrystalline cellulose and croscarmellose were sieved through an appropriate mesh size sieve and were mixed with the obtained granulate in a diffusion mixer. The rest of the magnesium stearate was sieved through an appropriate mesh size sieve and mixed with the granulate in a diffusion mixer. The homogeneous powder obtained, was compressed using a rotating tablet press using appropriate punches.

The tablets were coated with a water suspension of Opadry® II 31K28459 White coating until 3% weight gain (= 16.8 mg/140 mg tablet). The tablets were packed in suitable packaging material and were stored at 40°C/75% RH.

**Table 9: Stability results at 40°C/75% RH for coated tablets (composition C) prepared by dry granulation stored in different packaging materials**

| | | **t= 1 month 40°C/75% RH** | |
|---|---|---|---|
| **Test** | **t= 0** | **HDPE bottle/PP cap** | **Alu/Alu blister** |
| Assay (%) | 100.0 | 98.4 | 98.1 |
| Impurities (%) | | | |
| Impurity A | <0.05 | <0.05 | <0.05 |
| Impurity B | <0.05 | <0.05 | <0.05 |
| Impurity C | <0.05 | <0.05 | <0.05 |
| Impurity D | N.D. | N.D. | N.D. |
| Single largest unspecified | N.D. | 0.06 | <0.05 |
| Total unspecified impurities/ degradants | N.D. | 0.06 | <0.05 |
| **Total impurities/degradants** | **<0.05** | **0.06** | **<0.05** |

XRPD analysis performed after storing the tablets in several types of packaging material (HDPE open bottle, HDPE bottle/PP cap, Alu/Alu blister) for 1 month at 40°C/75% RH showed only reflections in accordance with anhydrous dasatinib.

## Claims

1. A tablet composition comprising anhydrous dasatinib and one or more pharmaceutically acceptable excipients, wherein the X-ray powder diffraction pattern of anhydrous dasatinib comprises characteristic peaks at the following 2 theta (± 0.2) angles: 6.8°, 11.1°, 12.3°, 13.2°, 13.7°, 16.7°, 21.0°, 24.3° and 24.8°, measured using a Cu Kα radiation and wherein the tablet is film coated with a coating composition comprising triacetin as plasticizer.

2. The tablet composition according to claim 1, wherein the coating composition further comprises a cellulose polymer and a pigment.

3. The tablet composition according to claim 2, wherein the cellulose polymer is hydroxypropylmethylcellulose.

4. The tablet composition according to claim 2 or 3, wherein the pigment is titanium dioxide.

5. The tablet composition according to any one of claims 1 to 4, wherein the coating composition further comprises lactose.

6. The tablet composition according to any one of claims 1 to 5 exhibiting a dissolution rate of at least 85% in 20 minutes when tested in aqueous hydrochloric acid 0.01 N in a USP apparatus II at 75 rpm, 25°C.

7. The tablet composition according to any one of claims 1 to 6, wherein the pharmaceutically acceptable excipients are chosen form one or more binders, diluents, disintegrants, glidants or lubricants.

8. A process for preparing the tablet composition according to any one of claims 1 to 7 comprising granulation.

9. The process according to claim 8 comprising wet granulation.

10. The process according to claim 8 comprising wet granulation using water as solvent.

11. The process according to claim 8 comprising dry granulation.

12. The tablet composition according to any one of claims 1 to 7 for use as a medicament.

13. The composition for use according to claim 12 in the treatment of chronic myeloid leukaemia (CML) and acute lymphoblastic leukaemia (ALL).

## Patentansprüche

1. Tablettenzusammensetzung, umfassend wasserfreies Dasatinib und einen oder mehrere pharmazeutisch annehmbare Trägerstoffe, wobei das Röntgen-Pulverbeugungsmuster von wasserfreiem Dasatinib charakteristische Spitzen an den folgenden 2 Theta (± 0.2)-Winkeln umfasst: 6.8°, 11.1°, 12.3°, 13.2°, 13.7°, 16.7°, 21.0°, 24.3° und 24.8°, gemessen unter Verwendung einer Cu-Ka-Strahlung, und wobei die Tablette mit einer Beschichtungszusammensetzung, die Triacetin als Weichmacher umfasst, filmbeschichtet ist.

2. Tablettenzusammensetzung nach Anspruch 1, wobei die Beschichtungszusammensetzung ferner ein Cellulosepolymer und ein Pigment umfasst.

3. Tablettenzusammensetzung nach Anspruch 2, wobei das Cellulosepolymer Hydroxypropylmethylcellulose ist.

4. Tablettenzusammensetzung nach Anspruch 2 oder 3, wobei das Pigment Titandioxid ist.

5. Tablettenzusammensetzung nach einem der Ansprüche 1 bis 4, wobei die Beschichtungszusammensetzung ferner Lactose umfasst.

6. Tablettenzusammensetzung nach einem der Ansprüche 1 bis 5, die eine Auflösungsrate von mindestens 85 % in 20 Minuten aufweist, wenn sie in wässriger Salzsäure 0.01 N in einer USP-Typ-II-Vorrichtung bei einer Umdrehungsrate von 75 rpm und bei 25 °C getestet wird.

7. Tablettenzusammensetzung nach einem der Ansprüche 1 bis 6, wobei die pharmazeutisch annehmbaren Trägerstoffe ausgewählt sind aus einem oder mehreren Bindemitteln, Verdünnungsmitteln, Sprengmitteln, Gleitmitteln oder Schmiermitteln.

8. Verfahren zur Herstellung einer Tablettenzusammensetzung nach einem der Ansprüche 1 bis 7, umfassend Granulierung.

9. Verfahren nach Anspruch 8, umfassend Nassgranulierung.

10. Verfahren nach Anspruch 8, umfassend Nassgranulierung unter Verwendung von Wasser als Lösungsmittel.

11. Verfahren nach Anspruch 8, umfassend eine Trockengranulierung.

12. Tablettenzusammensetzung nach einem der Ansprüche 1 bis 7 zur Verwendung als Medikament.

13. Zusammensetzung nach Anspruch 12 zur Verwendung bei der Behandlung von chronischer myeloischer Leukämie (CML) und akuter lymphoblastischer Leukämie (ALL).

## Revendications

1. Composition de comprimés comprenant du dasatinib anhydre et un ou plusieurs excipients pharmaceutiquement acceptables, dans lequel le schéma diffraction de poudre de rayons X du dasatinib anhydre comprend des pics caractéristiques aux angles de 2 theta (± 0.2) suivants : 6.8°, 11.1°, 12.3°, 13.2°, 13.7°, 16.7°, 21.0°, 24.3° et 24.8°, mesuré à l'aide d'un rayonnement Cu Kα et dans lequel le comprimé a une présentation pelliculée avec une composition d'enrobage comprenant de la triacétine comme plastifiant.

2. Composition de comprimé selon la revendication 1, dans laquelle la composition de l'enrobement comprend en outre un polymère de cellulose et un pigment.

3. Composition de comprimés selon la revendication 2, dans laquelle le polymère de cellulose est l'hydroxypropylméthylcellulose.

4. Composition de comprimés selon la revendication 2 ou 3, dans laquelle le pigment est le dioxyde de titane.

5. Composition de comprimés selon l'une quelconque des revendications 1 à 4, dans laquelle la composition de l'enrobage comprend en outre le lactose.

6. Composition de comprimés selon l'une quelconque des revendications 1 à 5 montrant un taux de dissolution d'au moins 85% en 20 minutes lorsqu'il est testé dans un acide chlorhydrique aqueux 0.01 N dans un appareil USP II à 75 tr/min à 25°c.

7. Composition de comprimés selon l'une quelconque des revendications 1 à 6, dans laquelle les excipients pharmaceutiquement acceptables sont choisis à partir d'un ou plusieurs liants, diluants, désintégrants, agents glissants ou lubrifiants.

8. Procédé de préparation de la composition de comprimés selon l'une quelconque des revendications 1 à 7 comprenant le procédé de granulation.

9. Procédé selon la revendication 8 comprenant la granulation humide.

10. Procédé selon la revendication 8 comprenant la granulation humide à l'eau comme solvant.

11. Procédé selon la revendication 8 comprenant la granulation sèche.

12. Composition de comprimés selon l'une quelconque des revendications 1 à 7, destinée à être utilisée comme médicament.

13. Composition selon la revendication 12 destinée à être utilisée dans le traitement de la leucémie myéloïde chronique (LMC) et de la leucémie lymphoblastique aiguë (LLA).
